(19) Europäisches Patentamt
European Patent Office
Office européen des brevets

(11) EP 1 772 461 A1

(12) **EUROPEAN PATENT APPLICATION**
published in accordance with Art. 158(3) EPC

(43) Date of publication:
**11.04.2007 Bulletin 2007/15**

(21) Application number: **05750924.2**

(22) Date of filing: **06.06.2005**

(51) Int Cl.:
***C07H 3/06*** *(2006.01)*

(86) International application number:
**PCT/JP2005/010316**

(87) International publication number:
**WO 2006/011301 (02.02.2006 Gazette 2006/05)**

(84) Designated Contracting States:
**DE FR GB**

(30) Priority: **29.07.2004 JP 2004221772**

(71) Applicants:
- **Ensuiko Sugar Refining Company, Limited Chuo-ku Tokyo, 103-0012 (JP)**
- **National University Corporation Tokyo University of Agriculture and Technology Fuchu-shi, Tokyo 183-8538 (JP)**

(72) Inventors:
- **MURAKAMI, Kazufumi Ensuiko Sugar Refining Co. Ltd. Yokohama-shi, Kanagawa 2360004 (JP)**
- **ITO, Tetsuya, Ensuiko Sugar Refining Co. Ltd.ama Yokohama-shi, Kanagawa 236-0004 (JP)**
- **FUJITA, Koki, Ensuiko Sugar Refining Co. Ltd.ohama Yokohama-shi, Kanagawa 236-0004 (JP)**
- **HARA, Kozo, Ensuiko Sugar Refining Co.,Ltd., Yokohama-shi, Kanagawa 236-0004 (JP)**
- **SAKANO, Yoshiyuki, NAC Tokyo University of A&T Fuchu-city, Tokyo 183-8538 (JP)**
- **KAMITORI, Shigehiro, Inf.Tech.Center, Kagawa Univ. Kita-gun Kagawa 761-0793 (JP)**

(74) Representative: **Gille Hrabal Struck Neidlein Prop Roos Brucknerstrasse 20 40593 Düsseldorf (DE)**

# (54) CRYSTALLINE LACTOSUCROSE OR MOLASSES-CONTAINING CRYSTAL HAVING THE SAME CONTAINED THEREIN, AND USE THEREOF

(57) The present invention establishes a crystalline lactosucrose and provides a process for producing the crystalline lactosucrose or a syrup containing crystalline lactosucrose. Further, the present invention has its object to provide a composition, for example, a food or drink, a cosmetic, a drug or the like containing the crystalline lactosucrose.

That is, the present invention provides the crystalline lactosucrose or the syrup containing crystalline lactosucrose, the process for producing the crystalline lactosucrose or the syrup containing crystalline lactosucrose, **characterized by** crystallizing a crystalline lactosucrose from a solution containing lactosucrose and collecting the crystalline lactosucrose, and the use thereof.

*FIG. 6*

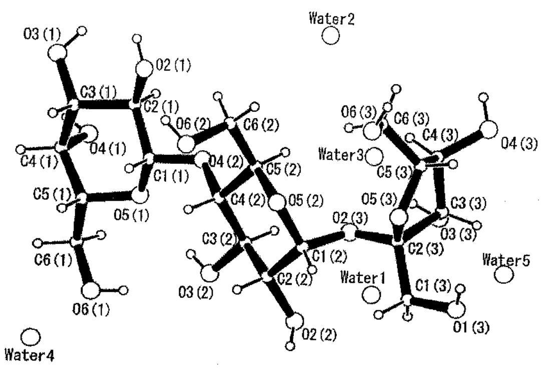

EP 1 772 461 A1

**Description**

Technical Field

The present invention relates to a novel crystalline carbohydrate, a process for producing the same and the use thereof, and more particularly relates to a crystalline lactosucrose or a syrup containing crystalline lactosucrose, a process for producing the same and the use thereof.

Background Art

Lactosucrose is a trisaccharide in which fructose is bound to the reducing end of lactose and is used in a variety of compositions such as a food and drink, a cosmetic and a drug.
This lactosucrose can be synthesized by the transfructosylation of β-fructofuranosidase using sucrose as a sugar donor and lactose as a sugar acceptor, however, the production rate of lactosucrose by this reaction is as low as about 40%. Accordingly, industrially, yeast is added to a reaction system to allow the yeast to assimilate the resulting byproduct glucose and the equilibrium reaction is allowed to shift to the side of production of lactosucrose thereby to increase the production rate thereof to about 60% (see Patent document 1).
However, in the case where lactosucrose of higher purity is intended to be obtained, it is necessary to perform separation and purification of lactosucrose using a reverse phase column chromatograph, a gel filtration chromatograph, a simulated moving bed ion chromatograph or the like, which is a cause of the high cost of production.
Accordingly, in order to solve the above problems, a method has been proposed, in which lactose, sucrose and fructosyl transferase are supplied to a simulated moving bed reactor, respectively, whereby lactosucrose of high purity is continuously obtained (see Patent document 2). However, there are problems that the reactor used at this time costs too much, control of the reaction is difficult and the like, and the practical application of the method has not been achieved yet.
Further, a powder product of lactosucrose has been produced by a known spray drying method. However, a product obtained by this method is unstable, i.e., it is very hygroscopic and is easily solidified and loses fluidity, therefore, establishment of a more stable crystal has been demanded.
Patent document 1: JP-A-4-293494
Patent document 2: JP-A-11-46788

Disclosure of the invention

Problems that the Invention is to Solve

A main object of the present invention is to establish a crystalline lactosucrose and to provide a process for producing the crystalline lactosucrose or a syrup containing crystalline lactosucrose.
The crystalline lactosucrose and the syrup containing crystalline lactosucrose as used herein may be any as long as they substantially have a crystalline property, and may be a crystalline lactosucrose of high purity or a syrup containing crystalline lactosucrose and a carbohydrate such as sucrose, lactose or kestose. Further, the purity of lactosucrose per solid content is not limited.

Means for Solving the Problems

In order to solve the above problem, the present inventors have made intensive studies. As a result, they found that when the purity of aqueous solution with a lactosucrose purity of about 60% was increased to 90 to 99% by subjecting to separation via chromatography such as ion chromatography, and the solution was concentrated to a lactosucrose concentration of 70 to 85 w/w%, and then the resulting lactosucrose solution was maintained at 10 to 35°C for several months, a crystal was deposited in the solution.
This crystal was added as a seed crystal to an aqueous solution at a lactosucrose concentration of 60 to 85 w/w%, preferably 78 w/w%, and crystallization was accelerated by gently stirring the solution. The resulting solution containing crystal was subjected to centrifugation. Then, the resultant crystal was washed by spraying a small amount of water on the crystal, whereby a crystal of high purity was collected.
When the physicochemical properties of this crystal were examined, they found that the crystal was a novel crystalline lactosucrose having low hygroscopicity, thus the present invention has been completed.
The invention according to claim 1 is a crystalline lactosucrose or a syrup containing crystalline lactosucrose. The invention according to claim 2 is the crystalline lactosucrose or the syrup containing crystalline lactosucrose according to claim 1, wherein the crystal is a hydrate.

The invention according to claim 3 is the crystalline lactosucrose or the syrup containing crystalline lactosucrose according to claim 1, wherein the crystal is a pentahydrate.
The invention according to claim 4 is the crystalline lactosucrose or the syrup containing crystalline lactosucrose according to claim 1, wherein the crystal is a monoclinic system.
The invention according to claim 5 is a process for producing a crystalline lactosucrose or a syrup containing crystalline lactosucrose, characterized by crystallizing a crystalline lactosucrose from a solution containing lactosucrose and collecting the crystalline lactosucrose.
The invention according to claim 6 is a composition characterized by containing a crystalline lactosucrose or a syrup containing crystalline lactosucrose.
The invention according to claim 7 is the composition according to claim 6, characterized in that the composition is a food or drink.
The invention according to claim 8 is the composition according to claim 6, characterized in that the composition is a cosmetic.
The invention according to claim 9 is the composition according to claim 6, characterized in that the composition is a drug or a quasi-drug.

Effect of the Invention

According to the present invention, a crystalline lactosucrose excellent in stability is provided. Further, according to a production process of the present invention, a crystalline lactosucrose of high purity can be easily produced on an industrial scale at low cost and high yield.
The crystalline lactosucrose obtained according to the present invention has low hygroscopicity and is more stable and easy to handle unlike a conventional powder product of lactosucrose. Therefore, the crystalline lactosucrose of the present invention can not only be advantageously used as a reagent or a chemical raw material, but also be advantageously used in various compositions such as a food and drink, a cosmetic and a drug. Thus, the present invention has a great effect on a practical use.

Brief Description of the Drawings

[Fig. 1] Fig. 1 is a view showing the hygroscopicity of a crystalline lactosucrose (at 25°C and a relative humidity of 33%).
[Fig. 2] Fig. 2 is a view showing the hygroscopicity of a crystalline lactosucrose (at 25°C and a relative humidity of 81%).
[Fig. 3] Fig. 3 is a photomicrograph of a crystalline lactosucrose.
[Fig. 4] Fig. 4 is a view showing the $^{13}C$-NMR spectrum of a crystalline lactosucrose.
[Fig. 5] Fig. 5 is a view showing the IR spectrum of a crystalline lactosucrose.
[Fig. 6] Fig. 6 is a view of the tertiary structure of a crystalline lactosucrose.

Best Mode for Carrying Out the Invention

Lactosucrose is a trisaccharide in which fructose is bound to the reducing end of lactose as shown by the following structural formula, and the degree of sweetness is about 30% of that of sugar and the calorific value is 1.7 kcal/g.
[Chemical 1]

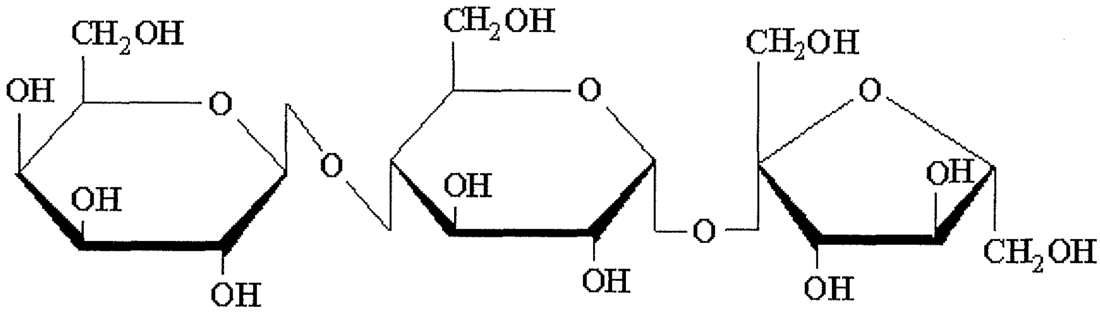

The crystalline lactosucrose and the syrup containing crystalline lactosucrose of the invention are crystals composed of the above lactosucrose. The crystal of lactosucrose may be a hydrate in which water is contained in the crystal lattice. As the hydrate, mono- to penta-hydrates are preferred, and among them, a pentahydrate is preferred. Further, the crystal system is not particularly limited, however, it is preferably a monoclinic system.

Hereinafter, a process for producing the crystalline lactosucrose and the syrup containing crystalline lactosucrose of the invention will be described.

A solution for depositing the crystalline lactosucrose of the invention may be any supersaturated lactosucrose solution so long as it can separate crystalline lactosucrose, and there is no limitation as to a process for producing lactosucrose. A specific example thereof is shown below. By using lactosucrose with a purity of about 30% or more, preferably 30 to 95%, an aqueous solution thereof at a concentration of 5 to 95 w/w%, preferably 60 to 85 w/w% is prepared, and the temperature of the solution may be set to any temperature at which the solution is not frozen and which is not higher than the melting point of the crystal with less possibility of browning or degradation of lactosucrose. For example, to a solution with a water content of about 5% of more, preferably 15 to 40%, a seed crystal is added at about 5 to 100°C, preferably at 20 to 25 °C to accelerate crystallization, whereby a solution containing crystal can be formed. Further, when a crystal is deposited, for the purpose of adjusting the degree of supersaturation or the viscosity of the solution, a water-soluble organic solvent such as methanol, ethanol or acetone may be optionally allowed to coexist therein.

The process for producing the crystalline lactosucrose and/or the syrup containing crystalline lactosucrose from the obtained solution containing crystal may be any as long as it is a method capable of collecting the crystalline lactosucrose and/or the syrup containing crystal, and a known method such as a syrup separation method, a spray drying method, a block pulverization method or a fluid granulating method can be employed. In particular, a basket-type centrifugal separator used in a syrup separation method is effective in the production of the crystalline lactosucrose, and it is also easy to wash the crystal by spraying a small amount of cold water, a cold alcohol solution or the like on the crystal as needed.

Further, with regard to a syrup containing crystal produced without completely separating syrup, the purity of lactosucrose is not so much improved, but the yield of the product is increased. In the case of this product, in addition to the crystalline lactosucrose, carbohydrates derived from the raw material such as sucrose and lactose are contained as syrup components.

The method other than the syrup separation method is effective in the production of the syrup containing crystal. In the case of the spray drying method, generally when a solution containing crystal with a concentration of 60 to 90%, preferably 70 to 75% and with a crystallization ratio of about 20 to 70%, preferably 40 to 50% is sprayed and dried at a temperature at which a crystal powder does not melt, a syrup containing crystal can be easily produced. Further, in the case of the block pulverization method, generally when a solution containing crystal with a water content of 5 to 15%, preferably 10 to 12% with a crystallization ratio of about 10 to 60%, preferably 40 to 50% is let stand for 1 to 5 days, preferably 2 to 3 days to solidify it into a block form and the block is pulverized and dried, a syrup containing crystal can be easily produced.

The crystalline lactosucrose and the syrup containing crystalline lactosucrose obtained in this way are easy to handle because they are substantially scarcely-hygroscopic although the hygroscopicity varies to some degree depending on the amount of the crystalline lactosucrose contained therein, and there is no concern for cohesion or caking. Therefore, a food or drink such as a powder sweetener, a chewing gum, a chocolate, an instant soup or a tablet, which was considered to be impossible to produce or extremely difficult to produce by using conventional lactosucrose, can be extremely easily and industrially produced. Further, other than a food and drink, they can be used in various purposes such as a cosmetic, a drug, a quasi-drug, a chemical raw material and a feed.

The composition according to claim 6 means the embodiments described above, however, it is not limited to these. The

form of the composition is arbitrary and may be in a form of a liquid, a powder or a granule, and other than these, a capsule, a tablet or another molded article. In the composition, a generally used component such as an excipient, a binder, a stabilizer, a filler, a lubricant, a disintegrant, a sweetening agent or a coloring agent can be appropriately blended as needed.

As described above, if the production process of the invention is employed, lactosucrose of high purity can be easily obtained from a carbohydrate mixture solution containing lactosucrose. Moreover, a fractionation step, and a spray drying step, a fluid granulating step or the like, which are the steps conventionally carried out, can be omitted, therefore, it is possible to produce lactosucrose at a low cost.

Examples

Hereinafter, the present invention will be described in detail with reference to Examples, however, the invention is not limited to these.
The sugar compositions in the following Examples were obtained by analyzing the respective samples by high performance liquid chromatography (HPLC) and performing calculation based on the peak areas. HPLC was carried out under the following conditions.

Column: TSK-GEL Amide-80 (manufactured by TOSOH Co., Ltd.) Mobile phase: 71% acetonitrile
Flow rate: 1.0 mL/min
Column temperature: 35°C
Detector: differential refractive index detector

(Example 1) Production of seed crystal of crystalline lactosucrose

After 1 part by weight of sucrose and 1 part by weight of lactose were dissolved by heating in 3 parts by weight of water, the solution was cooled to 37°C. Then, β-fructofuranosidase from Arthrobacter sp. K-1 (JP-A-3-27285) was added to the solution at a ratio of 7 units per g of sucrose, and yeast (manufactured by Oriental Yeast Co., Ltd.) was further added thereto at 2% or more per weight and was allowed to react for 30 hours. After completion of the reaction, an inactivation treatment was carried out at 90°C for 1 hour, diatomaceous earth filtration was carried out, and then desalting and decolorization were carried out with an ion exchange resin, whereby a syrup of lactosucrose with a purity of 58.5% was obtained.
The obtained syrup was passed through a stainless column with an inner diameter of 15 cm packed with a resin for fractionation (UBK-530, manufactured by Nippon Rensui Co., Ltd.), whereby a syrup of lactosucrose with a purity of 89.5% was obtained. The obtained syrup was concentrated to about 75 w/w%, and left in a 10-L glass flask at 10 to 15°C for several months, and as a result, a crystal of lactosucrose was deposited in the inner wall of the flask.
The resulting crystal was added as a seed crystal to an aqueous solution of lactosucrose at a concentration of 78 w/w% with a purity of 98%, and crystallization was accelerated by gently stirring the solution.
The obtained solution containing crystal was subjected to centrifugation using a basket-type centrifugal separator. Then, a small amount of distilled water was added to the resulting crystal to wash the crystal, whereby a crystalline lactosucrose with a purity of 99.9% was obtained.
The thus obtained crystalline lactosucrose is non-hygroscopic even when it is left in a room, and it can be advantageously used as a seed crystal.

(Example 2) Production of crystalline lactosucrose

To each of the carbohydrate mixture solutions with a solid content of 100 kg shown in Table 1, 100 g of the seed crystal obtained in Example 1 was added, and crystallization was carried out in a crystallizer while it was gently rotating. The resulting crystal was collected by a centrifugal separator and washed with cold water. This crystal was dried in a drier, and the purity and the recovery rate were calculated. The results are shown in Table 2.

[Table 1]

Table 1: Composition of carbohydrate mixture solution (%)

| carbohydrate mixture solution No. | LSF | LS | 1-Kes | Lac | Suc | Monosaccharides | Others |
|---|---|---|---|---|---|---|---|
| 1 | 0.2 | 98.1 | 0.1 | 1.0 | 0.2 | 0.4 | 0.0 |
| 2 | 3.7 | 90.1 | 3.2 | 0.7 | 1.5 | 0.1 | 0.7 |
| 3 | 3.3 | 74.4 | 2.4 | 7.5 | 8.6 | 2.9 | 0.9 |

(continued)

| carbohydrate mixture solution No. | LSF | LS | 1-Kes | Lac | Suc | Monosaccharides | Others |
|---|---|---|---|---|---|---|---|
| 4 | 5.4 | 65.2 | 2.9 | 10.5 | 12.6 | 2.5 | 0.9 |
| 5 | 3.7 | 55.1 | 2.8 | 13.6 | 18.9 | 3.7 | 2.2 |

LSF: fructosyllactosucrose
LS: lactosucrose
1-Kes: 1-kestose
Lac: lactose
Suc: Sucrose

[Table 2]

Table 2: Test results of crystallization of lactosucrose

| carbohydrate mixture solution No. | Crystallization time (hour) | Purity of crystal obtained (%) | Recovery rate (%) |
|---|---|---|---|
| 1 | 4 | 99.9 | 68 |
| 2 | 6 | 99.3 | 64 |
| 3 | 28 | 97.4 | 43 |
| 4 | 58 | 93.5 | 34 |
| 5 | 140 | 90.1 | 26 |

As is evident from the results shown in Table 2, when the purity of lactosucrose was lower, the crystallization time became longer and the recovery rate was decreased. However, it was found that when the purity of lactosucrose in the carbohydrate mixture solution was 55% or more, lactosucrose with a purity of 90% or more was able to be obtained by a single crystallization.

(Example 3) Hygroscopicity of crystal

About 3 g of each of the crystalline lactosucrose with a purity of 99.9% obtained in Example 2 and a lactosucrose powder product (manufactured by Ensuiko Sugar Refining Co., Ltd., LS-90P) was weighed out in a weighing bottle. Then, the samples were stored in a constant temperature and humidity chamber having a relative humidity of 33% or 81% at 25°C, and the change in the weight was measured. The results are shown in Fig. 1 and Fig. 2.
As evident from the results shown in Fig. 1 and Fig. 2, the crystalline lactosucrose showed lower hygroscopicity compared with the conventional powder product.

(Example 4) Physicochemical properties of crystal

When the physicochemical properties of the crystalline lactosucrose with a purity of 99.9% obtained in Example 2 were examined, they were as follows.

(1) Elemental analysis (as an anhydride)

C = 42.36%          H = 7.45%

(2) Mass analysis (as an anhydride)
MW = 510 (molecular formula: $C_{18}H_{38}O_{16}$)
(3) Water content (measured by the Karl-Fisher method)
15.2% (5 moles of water per mole of lactosucrose)
(4) Melting point

When 3 mg of the crystalline lactosucrose was placed in a dedicated aluminum container, and measurement was carried out with a differential scanning calorimeter, DSC-50 (manufactured by Shimadzu Co., Ltd.), endothermic peaks were detected between 53 and 55°C and between 173 and 175°C. It is considered that the endothermic peak

detected between 173 and 175°C appeared because the pentahydrate was converted to an anhydride while the temperature was raised. Accordingly, the melting point of the crystal is determined to be in the range of 53 and 55°C.

(5) Specific rotation

$$[\alpha]_D^{20} + 65.4°$$

(6) Ultraviolet absorption spectrum
When the substance is prepared as an aqueous solution and measurement is carried out, a characteristic absorption is not exhibited.

(7) Solubility

The solubility is high and about 220 g is dissolved in 100 mL of water at 25°C.

(8) Physical property, color and taste of the substance
It is a transparent and colorless crystal. A microcrystal is in a white powdery form and has a slight sweet taste, but does not have an odor. It is not hygroscopic or deliquescent. An example of the crystal when crystallization occurs from an aqueous solution of lactosucrose is shown in Fig. 3 by means of a photomicrograph.
(9) Color reaction
It exhibits green in the anthrone-sulfuric acid reaction, but is negative in the reaction by means of Fehling' s solution. It is negative in the iodine reaction.

(10) Structure

(a) When it is hydrolyzed with 1 N sulfuric acid, D-glucose, D-galactose and fructose are generated.
(b) By an action of β-fructofuranosidase, 1 mole of lactose and 1 mole of fructose were generated.
(c) The results of a carbon nuclear magnetic resonance analysis ($^{13}C$-NMR) are shown in Fig. 4.

(11) Infrared absorption spectrum
2 mg of a powder obtained by drying the crystal under reduced pressure at 80°C for 2 hours was stirred and mixed with 200 mg of dried KBr and a transparent tablet was prepared. Then, the infrared absorption spectrum was measured. The results are shown in Fig. 5.
(12) X-ray crystal structure analysis
A hydrated crystal (1.0 x 1.0 x 0.8 mm) crystallized from a supersaturated aqueous solution of lactosucrose (concentration of 75%) at 25°C was fixed to the tip of a glass rod with an epoxy resin adhesive, and diffraction data were collected at room temperature using a four circle auto-diffractometer (AFC7R) manufactured by Rigaku Corporation, and a rotating anode X-ray generator (RU300, output: 30 kV, 80 mA).
This crystal is a monoclinic system, and the space group was $P2_1$, the lattice constant was a = 11.099 Å, b = 14.453 Å, c = 8.361 Å, $\alpha$ = 90.0°, $\beta$ = 92.43° and $\gamma$ = 90.0°. Incidentally, a view of the tertiary structure of this crystal is shown in Fig. 6.
From the above properties, the crystal of the invention is determined to be an novel crystal of lactosucrose.

(Example 5) Production of mixed sweetener

In 1 part by weight of the crystalline lactosucrose with a purity of 99. 9% obtained by the method in Example 2, 0.1 part by weight of α-glycosyl stevioside (manufactured by Toyo Sugar Refining Co., Ltd., product name: α-G-sweet) was mixed to homogeneity, whereby a powder sweetener was produced. This product has a refined sweetness which is about 2 times that of sugar, and exhibits an effect of promoting the growth of Bifidobacteria.

(Example 6) Production of chewing gum

1 part by weight of gum base was subjected to a heat-melting treatment such that it became soft and was mixed with 3 parts by weight of the crystalline lactosucrose with a purity of 99. 9% obtained by the method in Example

2, 3 parts by weight of glucose and appropriate amounts of a mint flavor and a coloring agent. Then, the mixture was kneaded using a roll in accordance with a standard method and formed into a shape, whereby a chewing gum was produced. This product has a good texture and sweetness, and has an effect of promoting the growth of Bifidobacteria.

(Example 7) Production of hard candy

In 90 parts by weight of maltitol syrup (water content: 25 w/w), 15 parts by weight of the crystalline lactosucrose with a purity of 99.9% obtained by the method in Example 2 was dissolved by heating, and then the mixture was concentrated under reduced pressure until the water content became 2 w/w%. Subsequently, 0. 5 part by weight of citric acid and appropriate amounts of a lemon flavor and a coloring agent were mixed with the mixture, and then the mixture was formed into a shape in accordance with a standard method, whereby a hard candy was produced. This product is a hard candy, which has a refined sweetness and is low-cariogenic, and has an effect of promoting the growth of Bifidobacteria.

(Example 8) Production of chocolate

30 parts by weight of cacao paste, 10 parts by weight of cacao butter, 15 parts by weight of the crystalline lactosucrose with a purity of 99.9% obtained by the method in Example 2, 5 parts by weight of sucrose and 15 parts by weight of whole milk powder were mixed and passed through a refiner. After the grain size was reduced, the mixture was transferred into a conche and 0.5 part by weight of lecithin was added thereto, and then, the mixture was kneaded at 50°C for two days and nights.
Then, in accordance with a standard method, the mixture was poured into a molding machine, and formed into a shape and solidified, whereby a chocolate was produced. In this product, there is no fear of fat bloom or sugar bloom, and the way of melting in the mouth and the taste are both favorable. Further, this product exhibits an effect of promoting the growth of Bifidobacteria.

(Example 9) Production of milk-based drink

70 parts by weight of a liquid obtained by brewing coffee, 100 parts by weight of milk, 20 parts by weight of the crystalline lactosucrose with a purity of 99.9% obtained by the method in Example 2, and appropriate amounts of a coffee flavor and a coloring agent were mixed to homogeneity. Then, in accordance with a standard method, sterilizing, cooling, filling and packing were carried out, whereby a milk-based drink was produced. This product is a coffee milk drink having a good flavor and taste. Further, this product exhibits an effect of promoting the growth of Bifidobacteria.

(Example 10) Production of instant corn potage soup

25 parts by weight of gelatinized corn powder, 5 parts by weight of gelatinized starch, 3 parts by weight of gelatinized potato starch, 15 parts by weight of gelatinized waxy cornstarch, 8 parts by weight of the crystalline lactosucrose with a purity of 99.9% obtained by the method in Example 2, 5 parts by weight of sodium glutamate, 8 parts by weight of table salt, 7 parts by weight of powdered skim milk, and 0.5 part by weight of onion powder were pulverized and well mixed. Then, a substance obtained by heat-melting 0.5 part by weight of sorbitan fatty acid ester and 12 parts by weight of a hydrogenated plant oils was added thereto and mixed, and further 10 parts by weight of lactose was added thereto and mixed. Then, the mixture was fed to a fluidized-bed granulator, and granulation was carried out while spraying a small amount of water. Then, the granules were dried with hot air at 65°C and subjected to sieving, whereby an instant corn potage soup was produced. When hot water is poured on this product, the product is easily dissolved and dispersed to become a soup having a good flavor. Further, this product has an effect of promoting the growth of Bifidobacteria.

(Example 11) Production of custard cream

300 parts by weight of cornstarch, 500 parts by weight of the crystalline lactosucrose with a purity of 99.9% obtained by the method in Example 2, 300 parts by weight of maltose (distributed by Hayashibara Shoji, Inc. SUNMALT (registered trademark)), and 5 parts by weight of table salt were mixed well by passing them through a sieve, and then, 1500 parts by weight of chicken egg was added thereto and the mixture was stirred. Then, 5000 parts by weight of boiling milk was gradually added thereto, and the mixture was heated on a slow fire while stirring. The heating was stopped when the cornstarch was completely gelatinized and the mixture became translucent. Then, the mixture was cooled and an appropriate amount of a vanilla flavor was added thereto, whereby a custard cream was produced. This product has a smooth and glossy texture and has a good taste with no excessive sweetness. Further, this product has

an effect of promoting the growth of Bifidobacteria.

(Example 12) Production of nutritional supplement

A compound composed of 650 parts by weight of the crystalline lactosucrose with a purity of 99.9% obtained by the method in Example 2, 180 parts by weight of dried egg yolk, 210 parts by weight of powdered skim milk, 5 parts by weight of sodium chloride, 1.5 parts by weight of potassium chloride, 5 parts by weight of magnesium sulfate, 0.02 part by weight of thiamine, 0.1 part by weight of sodium ascorbate, 0.6 part by weight of vitamin E acetate and 0.04 part by weight of nicotinamide was prepared. 25 g each of this compound was packed into a laminated aluminum small bag and the bag was heat sealed, whereby a solid tube nutritional supplement was produced. In this product, the quality is stably maintained for a long time, and the solubility and the dispersibility are favorable. Further, this product has an effect of promoting the growth of Bifidobacteria.

(Example 13) Production of tablet

55 parts by weight of the crystalline lactosucrose with a purity of 99.9% obtained by the method in Example 2, 10 parts by weight of maltose, 1 part by weight of tricalcium phosphate, 1.5 parts by weight of a sugar ester and an appropriate amount of a powder flavor were mixed to homogeneity. Then, in accordance with a standard method, the mixture was tabletted with a tabletting machine such that one tablet weighed about 300 mg, whereby a tablet was obtained. This product is a tablet which does not have any crack and is stable and easy to swallow. By taking about 1 to 40 tablets, preferably about 2 to 20 tablets per adult, an effect of promoting the growth of Bifidobacteria and an effect of promoting the absorption of calcium are exhibited.

(Example 14) Production of compound feed

40 parts by weight of wheat gluten, 38 parts by weight of powdered skim milk, 15 parts by weight of the crystalline lactosucrose with a purity of 99.9% obtained by the method in Example 2, 10 parts by weight of a vitamin preparation, 4 parts by weight of fish powder, 5 parts by weight of dicalcium phosphate, 3 parts by weight of a liquid oil and fat, 5 parts by weight of calcium carbonate, 3 parts by weight of table salt and 2 parts by weight of a mineral preparation were mixed, whereby a compound feed was prepared. This product is a feed for livestock and poultry whose preference has been improved, and is particularly preferably as a feed for piglets. Further, this product exhibits an effect of growing Bifidobacteria and an effect of promoting minerals.

## Claims

1. A crystalline lactosucrose or a syrup containing crystalline lactosucrose.

2. The crystalline lactosucrose or the syrup containing crystalline lactosucrose according to claim 1, wherein the crystal is a hydrate.

3. The crystalline lactosucrose or the syrup containing crystalline lactosucrose according to claim 1, wherein the crystal is a pentahydrate.

4. The crystalline lactosucrose or the syrup containing crystalline lactosucrose according to claim 1, wherein the crystal is a monoclinic system.

5. A process for producing a crystalline lactosucrose or a syrup containing crystalline lactosucrose, **characterized by** crystallizing a crystalline lactosucrose from a solution containing lactosucrose and collecting the crystalline lactosucrose.

6. A composition **characterized by** containing a crystalline lactosucrose or a syrup containing crystalline lactosucrose.

7. The composition according to claim 6, **characterized in that** the composition is a food or drink.

8. The composition according to claim 6, **characterized in that** the composition is a cosmetic.

9. The composition according to claim 6, **characterized in that** the composition is a drug or a quasi-drug.

FIG. 1

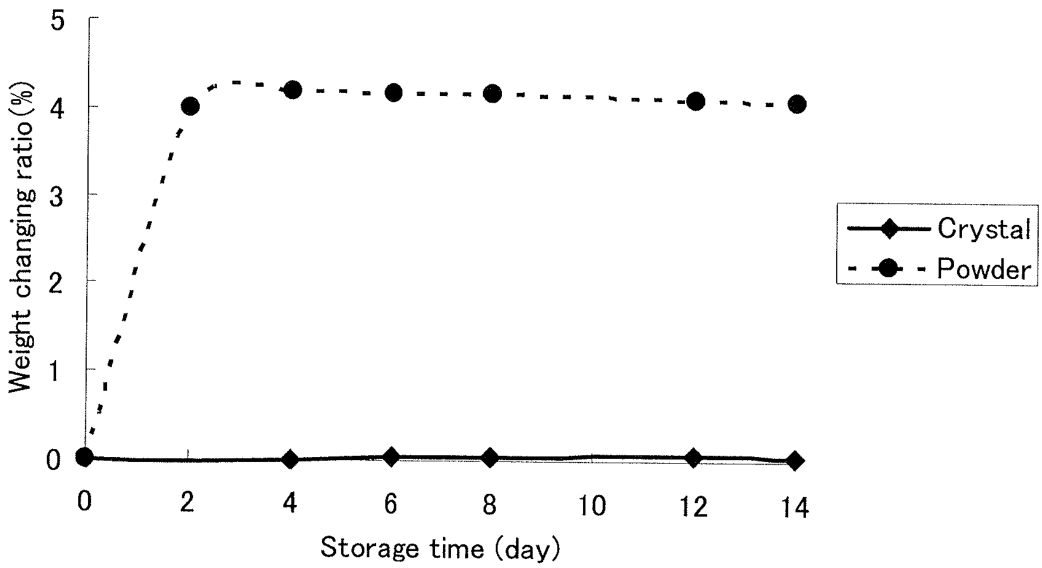
Weight changing ratio (%)
5
4
3
2
1
0
0
2
4
6
8
10
12
14
Storage time (day)
Crystal
Powder

FIG. 2

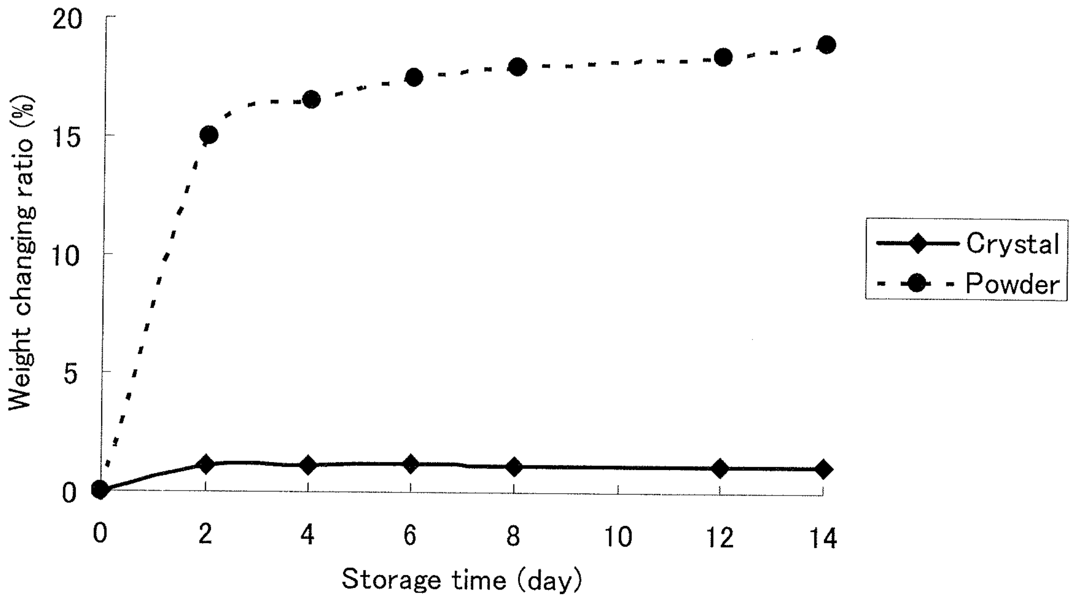
Weight changing ratio (%)
20
15
10
5
0
0
2
4
6
8
10
12
14
Storage time (day)
Crystal
Powder

FIG. 3

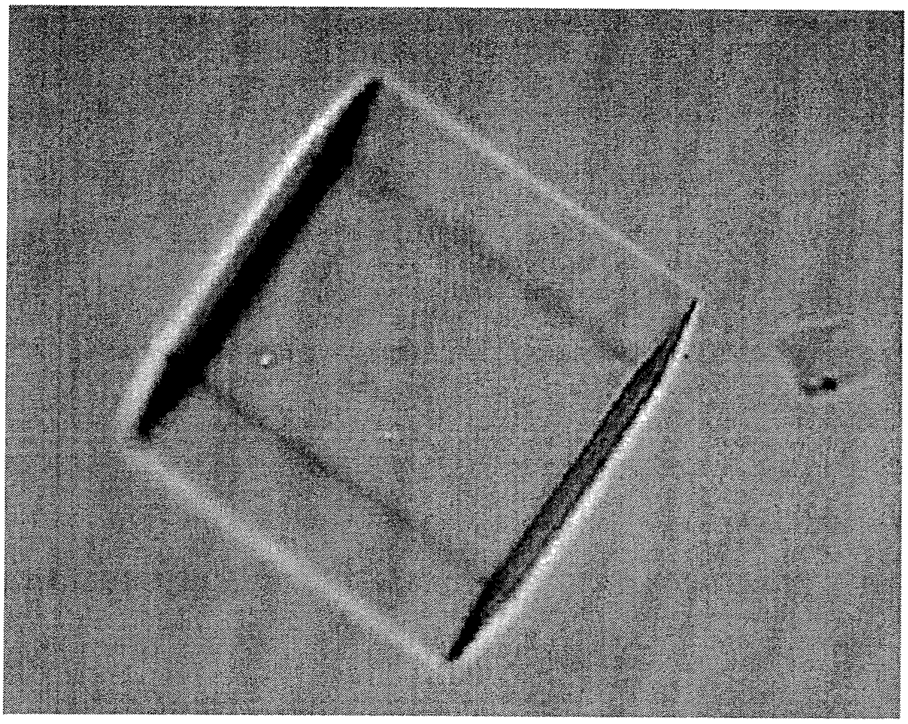

FIG. 4

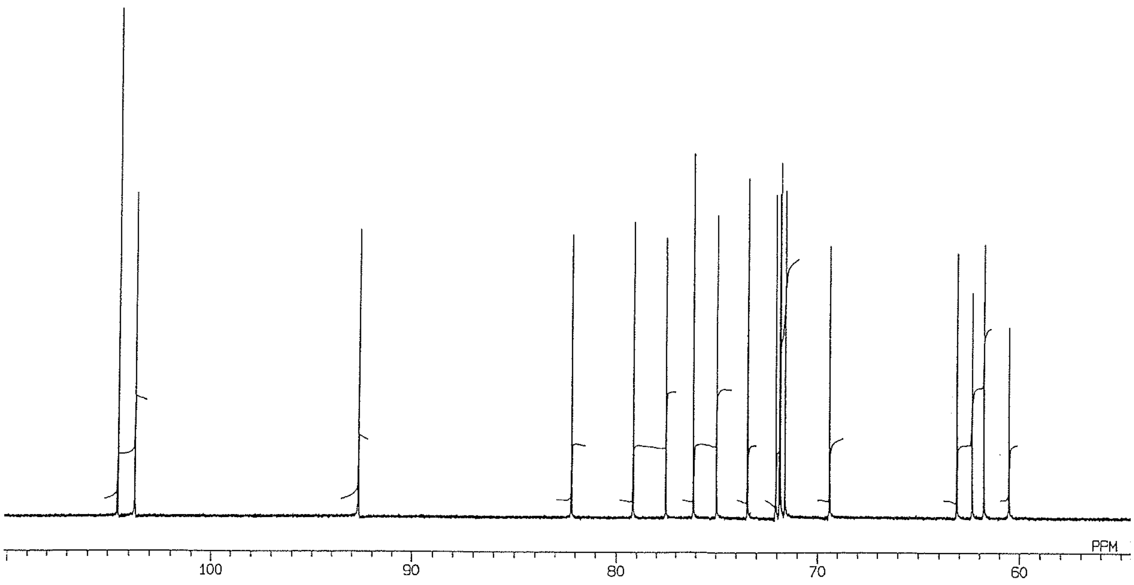

FIG. 5

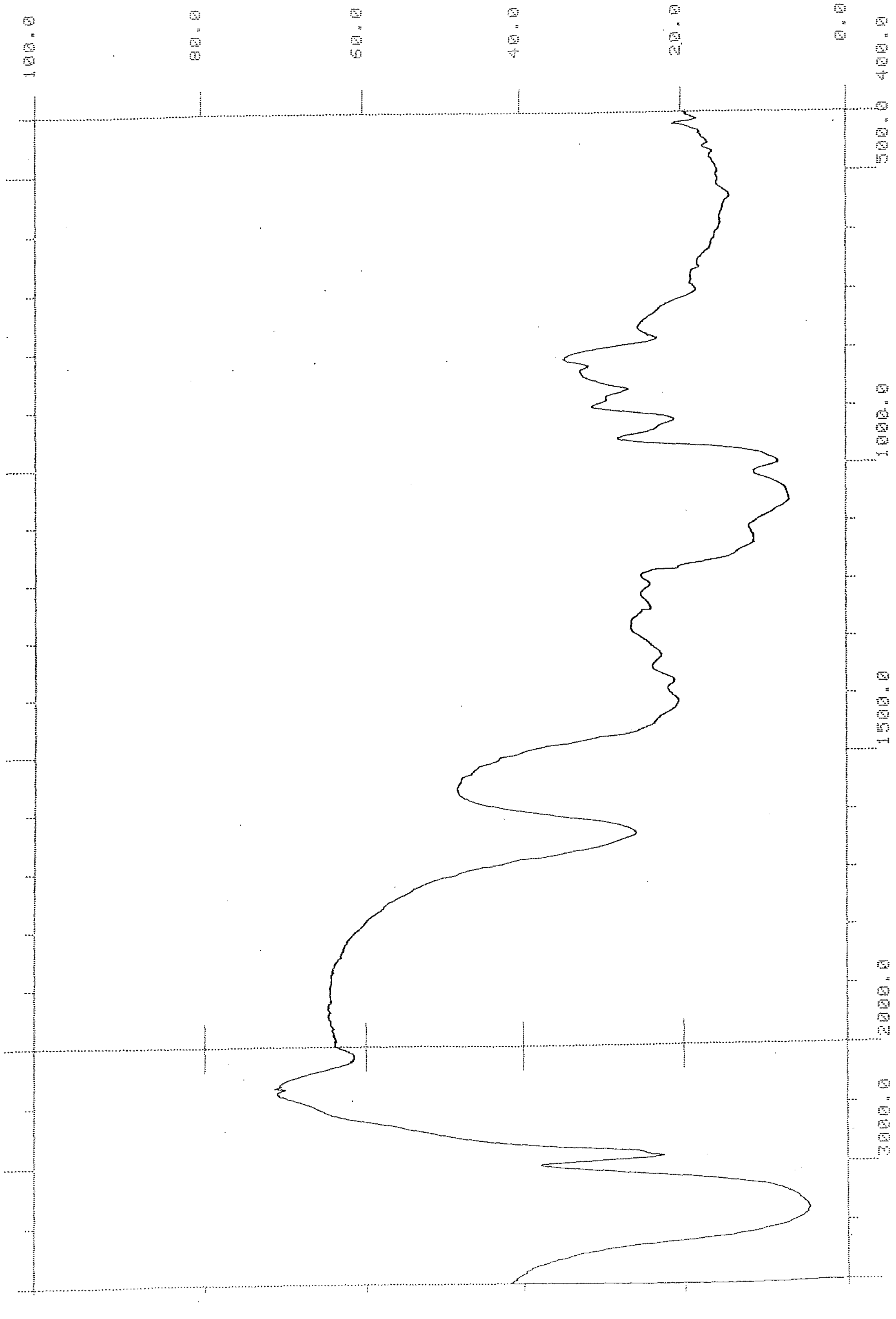

FIG. 6

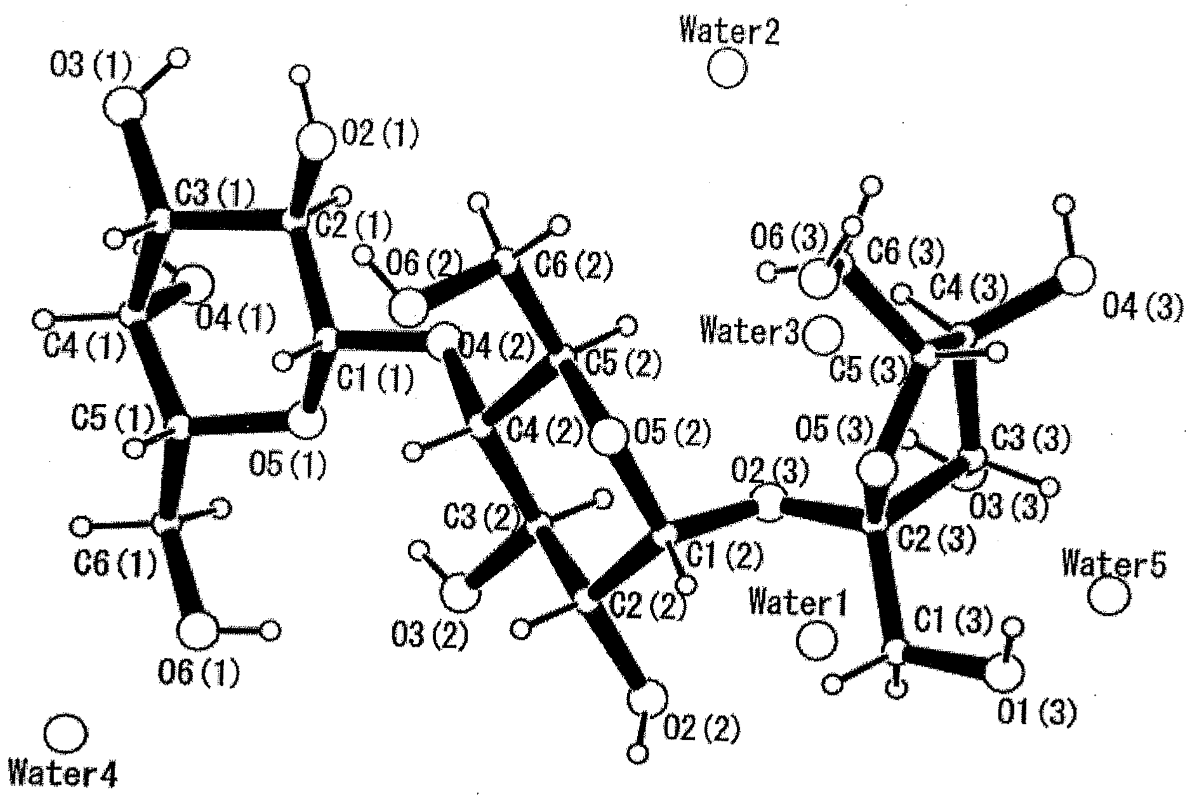

**INTERNATIONAL SEARCH REPORT**

International application No.
PCT/JP2005/010316

A. CLASSIFICATION OF SUBJECT MATTER
Int.Cl[7] C07H3/06, A23L1/09, A61K7/00, 31/702

According to International Patent Classification (IPC) or to both national classification and IPC

B. FIELDS SEARCHED

Minimum documentation searched (classification system followed by classification symbols)
Int.Cl[7] C07H3/06, A23L1/09, A61K7/00, 31/702

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAOLD(STN), CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 4-293494 A (Ensuiko Sugar Refining Co., Ltd.), 19 October, 1992 (19.10.92), Full text (Family: none) | 1-9 |
| Y | JP 10-57092 A (Kabushiki Kaisha Hayashibara Seibutsu Kagaku Kenkyujo), 03 March, 1998 (03.03.98), Full text & US 5130239 A & JP 4-281795 A | 1-9 |
| Y | JP 61-103889 A (Kabushiki Kaisha Hayashibara Seibutsu Kagaku Kenkyujo), 22 May, 1986 (22.05.86), Full text & US 4758660 A | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 August, 2005 (19.08.05) | 06 September, 2005 (06.09.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

INTERNATIONAL SEARCH REPORT

International application No.
PCT/JP2005/010316

C (Continuation). DOCUMENTS CONSIDERED TO BE RELEVANT

| Category* | Citation of document, with indication, where appropriate, of the relevant passages | Relevant to claim No. |
|---|---|---|
| Y | JP 56-97297 A (Tarules Development (N.A.) N.V.), 05 August, 1981 (05.08.81), Full text & US 4343934 A | 1-9 |
| A | FARKAS, Erzsebet et al., Regioselective synthesis of galactosylated tri- and tetrasaccharides by use of β-galactosidase from Bacillus circulans, Synthesis, 2003, (5), 699-706 | 1-9 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4293494 A
- JP 11046788 A
- JP 3027285 A